# EUROPEAN PATENT APPLICATION

(11) **EP 3 269 400 A1**
(43) Date of publication of application: **17.01.2018**
(21) Application number: 17181497.3
(22) Date of filing: 14.07.2017
(51) Int. Cl.: A61L 27/12, A61L 27/46

(54) **AMOPRHOUS HYALURONIC ACID-MAGNESIUM/CALCIUM POLYPHOSPHATE MICROPARTICLES FOR CARTILAGE REGENERATION AND REPAIR**

(30) Priority: 15.07.2016 GB 201612280
(71) Applicant: NanotecMARIN GmbH, 55128 Mainz (DE)
(72) Inventor: MÜLLER, Werner E.G., 55129 Mainz (DE)
(74) Representative: Krauss, Jan

(57) **Abstract**

This invention concerns a biomimetic material based on energy-rich amorphous magnesium polyphosphate (Mg-polyP) microparticles that enhance cartilage synthesis and regeneration. One preferred formulation of the inventive material is a hyaluronic acid-Mg/Ca-polyP paste that can be produced from a water-soluble salt of polyP and water-soluble hyaluronic acid in the presence of water-insoluble/nearly insoluble calcium carbonate. Surprisingly, the inventor found that this cartilage-like material comprising amorphous Mg/Ca-polyP microparticles promotes the adhesion of chondrocytes and strongly upregulates the expression of the chondrocyte marker genes encoding alkaline phosphatase, collagen type 3A1, aggrecan and Sox9. The material through scavenging calcium ions (Mg²⁺/Ca²⁺ exchange) and binding of the calcium-polyP to hyaluronic acid shows biomechanical properties, comparable to cartilage and thus can be used for prevention of calcium crystal formation in the synovial fluid and treatment of joint dysfunctions caused by osteoarthritis.

## Description

This invention concerns a biomimetic material based on energy-rich amorphous magnesium polyphosphate (Mg-polyP) microparticles that enhance cartilage synthesis and regeneration. One preferred formulation of the inventive material is a hyaluronic acid-Mg/Ca-polyP paste that can be produced from a water-soluble salt of polyP and water-soluble hyaluronic acid in the presence of water-insoluble/nearly insoluble calcium carbonate. Surprisingly, the inventor found that this cartilage-like material comprising amorphous Mg/Ca-polyP microparticles promotes the adhesion of chondrocytes and strongly upregulates the expression of the chondrocyte marker genes encoding alkaline phosphatase, collagen type 3A1, aggrecan and Sox9. The material through scavenging calcium ions (Mg²⁺/Ca²⁺ exchange) and binding of the calcium-polyP to hyaluronic acid shows biomechanical properties, comparable to cartilage and thus can be used for prevention of calcium crystal formation in the synovial fluid and treatment of joint dysfunctions caused by osteoarthritis.

### Background of the Invention

Osteoarthritis affects nearly 10% of the population worldwide and is the most common form of joint disease in older people (Helmick CG, et al. (2008) Estimates of the prevalence of arthritis and other rheumatic conditions in the United States. Part I. Arthritis Rheum 58: 15-25). The economic costs of this disease are extremely high. In the US, $185.5 billion insurer expenditures have been attributed to medical care for patients with osteoarthritis in 2009 (Kotlarz H, et al. (2009) Insurer and out-of-pocket costs of osteoarthritis in the US: evidence from national survey data. Arthritis Rheum 60: 3546-3553). In Europe, the annual costs of osteoarthritis per patient range from €1,330 to €10,452 (Hiligsmann M, Reginster JY (2013) The economic weight of osteoarthritis in Europe. Medicographia 35:197-202).

Osteoarthritis is characterized by a progressive loss of articular cartilage and chondrocytes within the synovial joints that causes chronic joint pain and functional limitations (Sharma AR, et al. (2013) Interplay between cartilage and subchondral bone contributing to pathogenesis of osteoarthritis. Int J Mol Sci 14: 19805-19830). In the synovial fluid and tissues of osteoarthritis patients, calcium crystals are frequently found that are composed of Ca-pyrophosphate and Ca-phosphate (Rosenthal AK (2011) Crystals, inflammation, and osteoarthritis. Curr Opin Rheumatol 23: 170-173). In addition, the expression of genes, involved in mineralization, e.g. the alkaline phosphatase (ALP), is upregulated in chondrocytes from osteoarthritis patients. These findings indicate that articular calcification, occurring during osteoarthritis, is associated with altered gene expression.

The osteoarthritis of the knee is classified as either primary or secondary. The hyaline joint cartilage which mainly consists of extracellular matrix and only sparsely distributed cells (chondrocytes) is the main target where the disease starts. This matrix is composed of water, aggrecan, collagen, and proteoglycans. Aggrecan, a proteoglycan, provides together with hyaluronic acid (HA) to the cartilage the proper hydrated gel structure and by that the required load-bearing properties. Different from most tissues, articular cartilage is not traversed by blood vessels, even though this tissue requires a considerable amount of energy (Ahlqvist J, Osterlund K, Harilainen A (1989) Does joint cartilage require energy? Ann Rheum Dis 48: 878). Glucose is considered as the major energy supply molecule for cartilage tissue, but this molecule has to enter the cells before it can provide energy in form of ATP.

ATP can be released by chondrocytes. This molecule has also been identified in the synovial fluid, and is considered to be a substrate for the production of inorganic pyrophosphate (Ryan LM, et al. (1991) Synovial fluid ATP: a potential substrate for the production of inorganic pyrophosphate. J Rheumatol 18: 716-720). However, since pyrophosphate is a substrate for ALP and ALP exists in the synovial fluid (Nanke Y, et al. (2002) Alkaline phosphatase in rheumatoid arthritis patients: possible contribution of bone-type ALP to the raised activities of ALP in rheumatoid arthritis patients. Clin Rheumatol 21: 198-202) it is doubtful that larger amounts of ATP are present in the extracellular space. Therefore, it remained open from where the metabolic energy, required for the maintenance of extracellular metabolism, the metabolic fuel, comes from.

The inventor provided experimental evidence that polyphosphate (polyP), which are abundantly present both intracellularly (e.g., within blood platelets) and extracellularly (Morrissey JH, et al. (2012) Polyphosphate: an ancient molecule that perhaps links platelets, coagulation, and inflammation. Blood 119: 5972-5979), could serve as an energy source for the generation of chemically utilizable Gibbs free energy after cleavage by ALP (reviewed in: Muller WEG, et al. (2015) Polyphosphate: a morphogenetically active implant material serving as metabolic fuel for bone regeneration. Macromolec Biosci 15: 1182-1197; Wang XH, et al. (2016) Polyphosphate as a metabolic fuel in Metazoa: A foundational breakthrough invention for biomedical applications. Biotechnol J 11: 11-30). Blood platelets exist in large amounts in synovial fluids from patients with rheumatoid arthritis and only in small quantities in fluids from osteoarthritis patients (Farr M, et al. (1984) Platelets in the synovial fluid of patients with rheumatoid arthritis. Rheumatol Int 4: 13-17). The inventor demonstrated that the synovial fluid is rich with respect to polyP. Another polymer that may ameliorate the pathophysiological symptoms of osteoarthritis is hyaluronic acid that acts as a potential joint lubrication (Akmal M, et al. (2005) The effects of hyaluronic acid on articular chondrocytes. J Bone Joint Surg Br 87: 1143-1149).

The following patent applications relate to polyP and respective uses: GB1406840.7. Morphogenetically active hydrogel for bioprinting of bioartificial tissue. Inventor: Müller WEG, Schröder HC, Wang XH. GB1403899.6. Synergistic composition comprising quercetin and polyphosphate for treatment of bone disorders. Inventor: Müller WEG, Schröder HC, Wang XH. GB1420363.2. Morphogenetically active calcium polyphosphate nanoparticles. Inventor: Muller WEG, and GB1502116.5. Synergistically acting amorphous calcium-polyphosphate nanospheres containing encapsulated retinol for therapeutic applications. Inventor: Müller WEG.

In cells, polyP is not present in the crystalline form but as an amorphous polymer (Morrissey JH, et al. (2012) Polyphosphate: an ancient molecule that links platelets, coagulation, and inflammation. Blood 119: 5972-5979). In order to develop a biomimetic approach for treatment of musculoskeletal disorders the inventor previously introduced a process that allows the preparation of amorphous microparticles of polyP (Muller WEG, et al. (2015) A new polyphosphate calcium material with morphogenetic activity. Materials Letters 148: 163-166). These particles have been prepared with Ca²⁺ as the counterion that was found also to self-organize and harden gel-like polyP after bioprinting (Muller WEG, et al. (2015) A new printable and durable N,O-carboxymethyl chitosan-Ca2+-polyphosphate complex with morphogenetic activity. J Mat Chem B 3: 1722-1730).

It is an object of the present invention, to provide an improved composition for the treatment of joint dysfunctions caused by osteoarthritis by preventing calcium crystal formation in the synovial fluid. Other objects of the present invention will become apparent when studying the following detailed description thereof.

In the present invention, the inventor prepared amorphous polyP microparticles with Mg²⁺ as counterions. The aim was to develop a strategy for the injection of polyP into the synovial fluid in order to lower the Ca²⁺-concentration in the fluid and to prevent the formation of Ca²⁺-crystals; the concept underlying this invention was that Mg-polyP will chelex out Ca²⁺ from the synovial fluid and bind this cation to the microparticles. Furthermore, Mg²⁺ has the potency to retard the nucleation and growth of Ca-phosphate crystals (Nabiyouni M, et al. (2015) Magnesium substitution in the structure of orthopedic nanoparticles: A comparison between amorphous magnesium phosphates, calcium magnesium phosphates, and hydroxyapatites. Mater Sci Eng C Mater Biol Appl 52: 11-17). Hyaluronic acid is an anionic polymer that can bind Ca²⁺ even if incorporated into crystal particles (Lamontagne CA, et al. (2011) Characterization of hyaluronic acid interaction with calcium oxalate crystals: implication of crystals faces, pH and citrate. J Mol Recognit 24: 733-740). In addition, hyaluronic acid is present in considerable amount (3-4 mg/ml) in the synovial fluid (Nitzan DW, et al. (2001) The role of hyaluronic acid in protecting surface-active phospholipids from lysis by exogenous phospholipase A2. Rheumatology (Oxford) 40: 336-340). Therefore, the inventor developed a strategy that uses Mg-polyP microparticles as active components in a potential injection fluid that is able to bind Ca²⁺ (from the synovial fluid) which in turn undergoes binding of polyP to hyaluronic acid under formation of a small lubrication film that could bind to the cartilage surface.

Surprisingly, the inventor found that Mg-polyP microparticles, in the presence of hyaluronic acid, potentiate the gene expression of *collagen type 3A1* as well as the expression of the transcription factor *Sox9.* Both proteins are involved in the differentiation of chondrocytes during cartilage repair (Salminen H, et al. (2001) Expression of Sox9 and type IIA procollagen during attempted repair of articular cartilage damage in a transgenic mouse model of osteoarthritis. Arthritis Rheum 44: 947-955). Based on the results underlying this invention it can be concluded for the first time that in the synovial fluid, which contains polyP, besides of hyaluronic acid (Decker B, et al. (1959) Concentration of hyaluronic acid in synovial fluid. Clinical Chemistry 5: 465-469) and Ca²⁺ [see Examples, below], also the anionic, nonsulfated glycosaminoglycan interacts in the fluid with the anionic polyP *via* divalent cations, e.g. Mg²⁺ or Ca²⁺.

Another important aspect of the invention is the provision of the formulation for an artificial cartilage material based on Mg-polyP that, together with hyaluronic acid, biomimics cartilage and comprises the advantageous property of the potential implant for an application *in vivo* to readily incorporate Ca²⁺; this cation is abundantly present in the synovial fluid especially after cartilage damage and bone hydroxyapatite disintegration (Wan LQ, et al. (2008) Calcium concentration effects on the mechanical and biochemical properties of chondrocyte-alginate constructs. Cell Mol Bioeng 1: 93-102). The new material shows biomechanical properties, comparable to cartilage, and elicits morphogenetic activity in chondrocytes through upregulation of the genes encoding for ALP, collagen 3A1 (COL3A1) and aggrecan. ALP is an essential enzyme in both hard and soft tissues, like in cartilage, the fibrillar collagen 3A1 is likewise present in cartilage; its gene is induced in chondrocytes undergoing a healing process. Aggrecan is a large proteoglycan that tightens articular cartilage and provides this tissue with the property to withstand compressive loads.

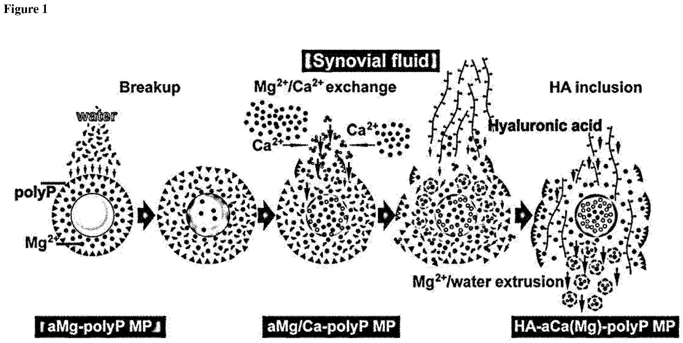

The synovial fluid surrounding the articular cartilage contains by far more Ca²⁺, compared to Na⁺ or Mg²⁺. An increased Ca²⁺ level in the synovial fluid has been implicated in the etiology of the calcium phosphate crystal formation frequently found in osteoarthritis patients (Yavorskyy A, et al. (2008) Detection of calcium phosphate crystals in the joint fluid of patients with osteoarthritis - analytical approaches and challenges. Analyst 133: 302-318). The inventive material can be used for for drawing out free Ca²⁺ from the synovial fluid (see scheme in Figure 1).

In addition, the anionic polymer, hyaluronic acid, abundantly exists in the synovial fluid (Balazs EA, et al. (1967) Hyaluronic acid in synovial fluid. I. Molecular parameters of hyaluronic acid in normal and arthritis human fluids. Arthritis Rheum 10: 357-376). According to this invention, after injection into the synovial fluid, the inventive Mg-polyP microparticles will be rebuilt to amorphous hyaluronic acid-Ca-polyP microparticles (see also Figure 1). To rebuild this process biomimetically, polyP as a Ca²⁺-binding polymer and a metabolic energy (fuel) source, the inventor produced Mg-polyP together with hyaluronic acid, in the presence of small amount of the highly water-insoluble CaCO₃ (solubility at 25°C at 13 mg/L). With time (after about 20 min) the exchange reaction Mg²⁺ by Ca²⁺ was terminated (HA-aMg/Ca-polyP-p). The resulting material has a consistency of a paste and consists of 77% water, a value that matches the content in human cartilage (Amadò R, et al. (1976) Water content of human articular cartilage and its determination by gas chromatography. Biochem Med 16: 169-172).

A further aspect of this invention is based on the finding that the inventive material is prone to enzymatic hydrolysis by ALP, indicating that also the metabolic energy stored in the phosphoanhydride bonds of polyP and liberated after hydrolysis is released.

### Detailed description of the invention

The inventor provides a novel polyphosphate (polyP) material that can be used for the prevention and treatment of osteoarthritis, and related cartilage diseases that are exacerbated by the undesired formation of calcium crystals. The inventive material is characterized by the following properties:
a) The material is amorphous (non-crystalline);
b) The material comprises or consists of Mg²⁺-polyP microparticles having a diameter in the rage of about 100 to 500 nm;
c) The material forms a viscous paste in the presence of hyaluronic acid, a major component of the synovial fluid;
d) The material promotes the adhesion of chondrocytes; and
e) The material is morphogenetically active, for example induces the expression of the genes encoding collagen type 3A1 (marker for chondrocyte differentiation), and Sox9 (transcription factor that regulates chondrocyte differentiation and proliferation); this stimulatory effect of the material can be abolished by incubation with the polyP degrading ALP;

Similar microparticles can be obtained after addition of Mg²⁺ ions to a solution containing hyaluronic acid and soluble Na-polyP.

According to a first aspect thereof, the invention relates to a method for producing a pharmaceutical composition comprising amorphous magnesium polyphosphate microparticles, comprising the steps of i) dissolving a soluble polyphosphate salt in an aqueous medium, ii) adding an aqueous solution of a soluble magnesium salt to said polyphosphate salt solution, and iii) collecting and drying of the particles formed after washing with alcohol, such as ethanol.

According to a second aspect thereof, the invention relates to a method for producing a pharmaceutical composition comprising amorphous magnesium polyphosphate microparticles and hyaluronic acid, comprising the steps of i) mixing of an aqueous solution of a soluble polyphosphate salt with an aqueous solution of hyaluronic acid, ii) adding a soluble magnesium salt to the mixture from step (i), and iii) allowing said mixture to fully gelatinate.

Preferred is a method according to the invention, wherein said soluble polyphosphate salt is sodium polyphosphate. Preferred is the method according to the invention, wherein the chain length of said polyphosphate is between about 3 to about 1000 phosphate units, preferably between about 10 to about 100 phosphate units, and most preferred about 40 phosphate units.

Preferably, said magnesium salt is magnesium chloride.

Preferred is a method according to the invention, wherein the amorphous magnesium polyphosphate microparticles are formed from sodium polyphosphate in the presence of magnesium chloride at a stoichiometric ratio of 0.1 to 20 (phosphate to magnesium), preferably of 1 to 4, and most preferably of 2.

Preferred is a method according to the invention, wherein the magnesium polyphosphate material is obtained by addition of a solution containing between about 100 to 200 g of magnesium chloride hexahydrate to a solution containing between about 20 to 60 g/L of sodium polyphosphate at a ratio of approximately 1:1 (volume/volume; magnesium chloride solution to sodium polyphosphate solution).

According to a third aspect thereof, the invention relates to a method according to the invention, comprising i) admixing of about one volume of saline supplemented with about 10% [w/v] hyaluronic acid with about one volume of saline containing about 10% [w/v] Na-polyP, ii) adding about two volumes of magnesium chloride hexahydrate (at 50 mg/ml in saline), in order to initiate the gelation/ionic cross-linking reaction, and iii) allowing the obtained mixture to assure complete gelation.

Preferred is a method according to the invention, further comprising the addition of a water-insoluble calcium salt or a calcium salt with low solubility in water to the mixture from step (i) before the addition of the magnesium salt, and continuation of mixing in order to allow for a hyaluronic acid-magnesium/calcium-polyphosphate paste formation.

Preferably, the calcium salt is calcium carbonate.

Preferred is a method according to the invention, wherein the production of the cartilage-like material comprises i) preparing of an aqueous solution containing about 100 g/L of hyaluronic acid, ii) adding about 100 g of solid sodium polyphosphate to about 1 L of the solution prepared in step (i), iii) adding of about 50 g of solid calcium carbonate to about 1 L of the solution obtained in step (ii), iv) adding of about 150 g of solid magnesium chloride hexahydrate, dissolved in about 500 ml of distilled water, to the viscous hyaluronic acid/calcium carbonate/polyphosphate suspension obtained in step (iii), and v) continuous mixing for about 2 h allowing for a hyaluronic acid-magnesium/calcium-polyphosphate paste formation.

Another aspect of the present invention relates to a pharmaceutical composition comprising amorphous magnesium polyphosphate microparticles, a gel, and/or a paste produced according to a method according to the invention for use in medicine. Preferred is a use in the regeneration and repair of cartilage or cartilage-like bone material, or as a bonding material for cartilage and bone, or a use in the prevention and/or treatment of joint dysfunctions and osteoarthritis through scavenging of synovial fluid calcium ions implicated in crystal formation.

According to one embodiment, the inventive material (amorphous Mg-phosphate microparticles; abbreviated: aMg-polyP-MP) can be prepared according to this invention as follows:
a) Dissolution of a soluble polyP salt in an aqueous medium, such as water,
b) Addition, preferably slow addition, of an aqueous (e.g. saline) solution of a magnesium salt to said polyP salt solution, and
c) Collecting and drying of the particles as formed after washing with a suitable solvent, such as ethanol. The procedure can be performed at room temperature.

As a preferred example, the above method can be carried out using sodium polyP as a polyP salt (chain length: about 40 phosphate units) and magnesium chloride as a magnesium salt as follows.
a) Dissolution of about 1 g of Na-polyP in about 25 ml of distilled water (room temperature),
b) Slow, dropwise addition of an aqueous (e.g. saline) solution of 3.86 g of magnesium chloride hexahydrate (MgCl₂ • 6H₂O) in 25 ml to said Na-polyP solution (room temperature),
c) Stirring of the suspension as formed for an extended period of time, e.g. for 4 h, and
d) Collection of the particles as formed, and washing them with ethanol while filtering (0.45 µm filter; e.g., Nalgene Rapid-Flow), and drying at about 50°C.

Another preferred aspect of this invention is a method for the production of amorphous Mg²⁺-polyP microparticles in the presence of hyaluronic acid, comprising
a) Mixing of one volume of saline supplemented with about 10% [w/v] hyaluronic acid with one volume of saline containing about 10% [w/v] Na-polyP,
b) Adding of about 2 volumes of MgCl₂ • 6H₂O (50 mg/ml saline), through which the gelation/ionic cross-linking reaction is initiated, and
c) Gelating of the obtained HA-hydrogel, Mg²⁺-Na-polyP, for about 1 h in order to assure complete gelation (at room temperature).

In the context of the present invention, the term "about" shall mean +/- 10% of the value as indicated.

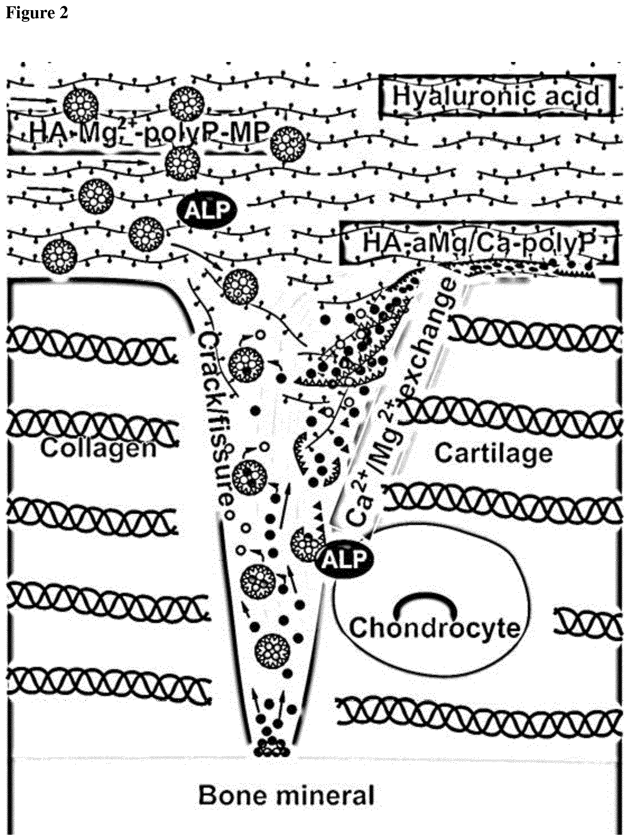

The inventive material can be used for treatment of joint dysfunctions caused by osteoarthritis through prevention of calcium crystal formation in the synovial fluid *via* scavenging Ca²⁺ ions (Mg²⁺/Ca²⁺ exchange) and enhancing chondrocyte function after binding of the Ca²⁺-polyP to hyaluronic acid at the cartilage surface. Due to the high propensity of the inventive amorphous Mg²⁺-polyP microparticles to de-assemble through opening of the ionic bonds in aqueous solution, they allow Ca²⁺ to form stronger and more durable polyP particles. The dynamic exchange of Mg²⁺ by Ca²⁺ can be supported by ALP, released by the chondrocytes. Since hyaluronic acid exposes, like polyP, anionic groups which can cross-link with polyP *via* Ca²⁺, the two polymers, polyP and hyaluronic acid, can form a new cartilaginous layer (see Figure 2). In complex with the inventive material, hyaluronic acid is protected against an enzymatic degradation by hyaluronidase present in the synovial fluid.

Another aspect of this invention then is an artificial cartilage-like material based on a hyaluronic acid-Mg/Ca-polyP material, such as a paste (HA-aMg/Ca-polyP-p), that is produced from the water-soluble Na-salt of energy-rich polyP and soluble hyaluronic acid in the presence of water-insoluble (nearly insoluble) calcium carbonate (CaCO₃).

As an example, the material can be produced according to a method using Na-polyP (chain length: 40 phosphate units) as a polyP salt as follows.
a) Dissolution of about 1 g of hyaluronic acid in about 10 ml of distilled water,
b) Addition of about 1 g of solid Na-polyP,
c) Addition of about 0.5 g of solid CaCO₃,
d) Addition of about 1.5 g of solid MgCl₂ • 6H₂O in about 5 ml of distilled water to the resulting viscous hyaluronic acid/CaCO₃/polyP suspension, and
e) Continuation of mixing for a suitable amount of time (about 2 h) allowing for the formation of a white paste (HA-aMg/Ca-polyP-p).

The inventive material (HA-aMg/Ca-polyP-p), after conversion of Na-polyP into the less soluble Mg/Ca-salt consisting of amorphous Mg/Ca-polyP microparticles, is able to mimic the physiological cartilage tissue and to bind Ca²⁺ ions present in the synovial fluid.

This material shows the following advantageous properties:
1. After the Mg²⁺/Ca²⁺ exchange and water extrusion the polyP component of this material becomes more stable, but is still prone to hydrolytic cleavage by ALP;
2. The material exhibits biomechanical properties, comparable to cartilage. Treatment with CaCl₂ resulted in an increase of the hardness (Young's modulus) as determined from 1.27 MPa to 3.23 MPa. In addition, the CaCl₂-treated material showed a faster stress increase, an almost complete lack of micro-crumblings and an extended creep period and elastic/viscoelastic recovery period;
3. The material exhibits morphogenetic activity as determined through upregulation of the chondrocyte marker genes encoding for ALP, collagen 3A1 and aggrecan.

The inventive material can be used for the treatment of osteoarthritis, cartilage repair, scavenging of synovial fluid calcium ions implicated in crystal formation in osteoarthritis patients, and as a bonding material for cartilage and bone.

The chain lengths of the polyP molecules can be in the range of between about 3 to about 1000 phosphate units, preferably about 10 to about 500, more preferred about 20 to about 100. Optimal results were achieved with polyP molecules with an average chain length of about 40 phosphate units.

A further preferred aspect of the invention relates to a material as obtained by one of the methods as described above. The inventive method can be used for the preparation of amorphous morphogenetically active polyphosphate microparticles. A further preferred aspect of the invention concerns a material containing such microparticles obtained by one of the methods described above. The technology according to this invention can be used for the production of microparticles or a material containing such microparticles to be used in regeneration and repair of cartilage.

Another aspect of the present invention relates to a method for regenerating and repairing of cartilage or cartilage-like bone material, or bonding cartilage and bone, comprising administering to a subject in need thereof a pharmaceutical composition according to the invention comprising amorphous magnesium polyphosphate microparticles, a gel, and/or a paste. Preferred is a method for the prevention and/or treatment of joint dysfunctions and osteoarthritis through scavenging of synovial fluid calcium ions implicated in crystal formation.

The invention will now be described further in the following more detailed examples, nevertheless, without being limited thereto. For the purposes of the present invention, all references as cited herein are incorporated by reference in their entireties.
**Figure 1** shows a schematic outline of the changes of amorphous Mg-polyP microparticles (aMg-polyP MP) undergoing in the synovial fluid. It is depicted that the microparticles formed of Mg-polyP readily dissolve partially and allow the Ca²⁺ ions as well as the hyaluronic acid (HA) polyanion, both present in the synovial fluid to bind to the polyanion polyP. After the exchange of Mg²⁺ by Ca²⁺ and inclusion of the hyaluronic acid polymers into the polyP core HA-aCa-polyP microparticles are formed; they surely contain still remaining Mg²⁺.
**Figure 2** shows the proposed processes of aMg-polyP-MP (or HA-Mg²⁺-Na-polyP) particles after injection into the synovial fluid space. Due to the relatively high propensity of the Mg-polyP-based particles (Mg²⁺-polyP) to open the ionic bonds and exposing the anionic groups to the aqueous milieu the physiological polymer polyP can undergo a new salt bridge with Ca²⁺ (Ca²⁺-polyP) [Ca²⁺/Mg²⁺ exchange]. During this process, the polymer can also cross-link with hyaluronic acid (HA). It is expected that HA-Mg/Ca-polyP can form an artificial layer on the surface of the cartilage and might close cracks and fissures. The chondrocytes can release the enzyme ALP that can partially hydrolyze the polyP chains.

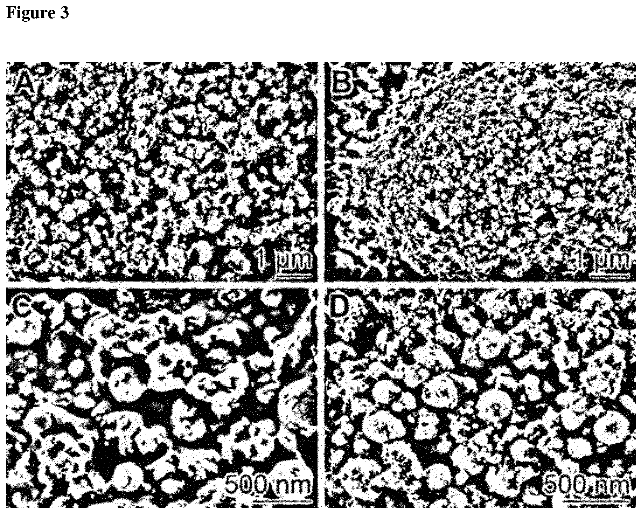

**Figure 3** shows the morphology of the amorphous Ca-polyP microparticles (aCa-polyP-MP) (**A** and **C)** and the Mg-polyP particles (aMg-polyP-MP) **(B** and **D);** SEM.

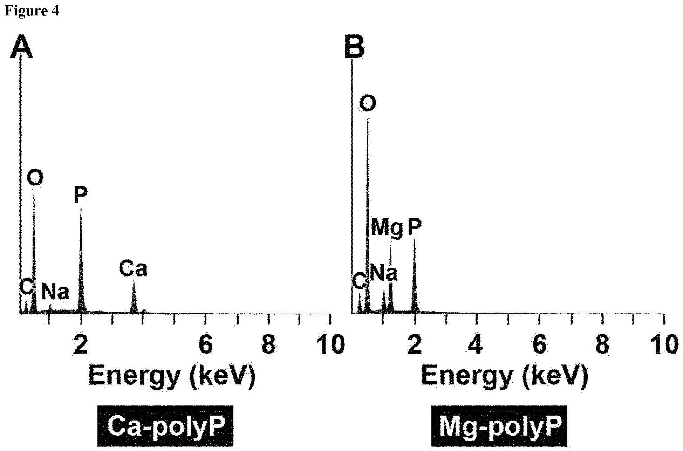

**Figure 4** shows the EDX spectra of aCa-polyP-MP (**A**) and aMg-polyP-MP **(B).** The signals for the elements C, O, Na, Mg, P and Ca are marked.

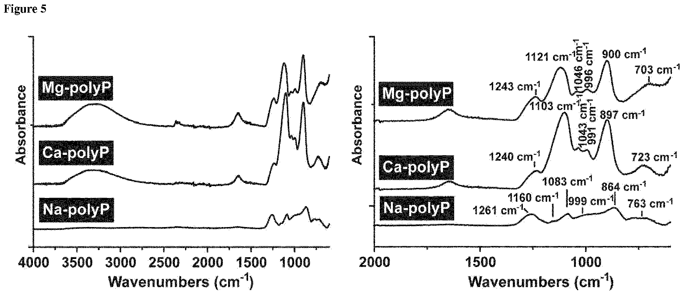

**Figure 5** shows the FTIR spectra of Na-polyP, Ca-polyP and Mg-polyP as recorded between the wavenumbers 4000 cm⁻¹ and 700 cm⁻¹ **(A);** in **(B)** an enlargement of the range between 2000 cm⁻¹ and 700 cm⁻¹ is given.

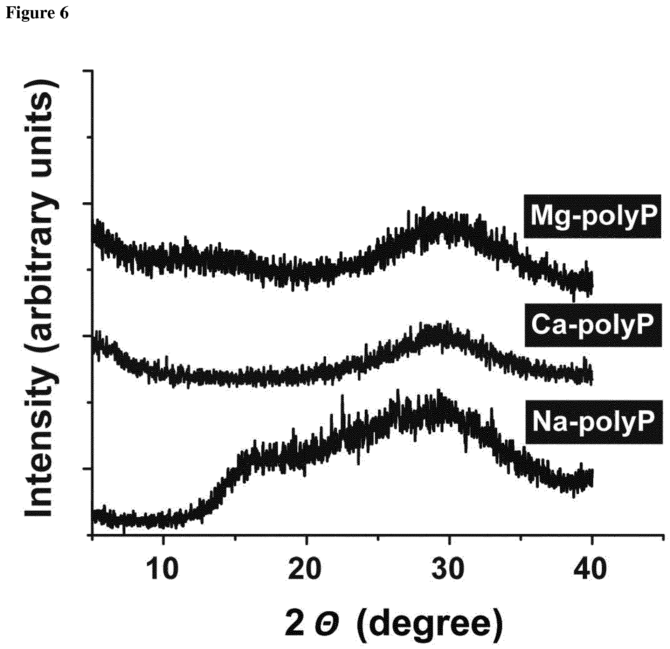

**Figure 6** shows the XRD pattern obtained for both the unchanged Na-polyP as well as the microparticles prepared from Ca-polyP and from Mg-polyP. No characteristic signals are detected that would indicate some form of crystalline polyP material.

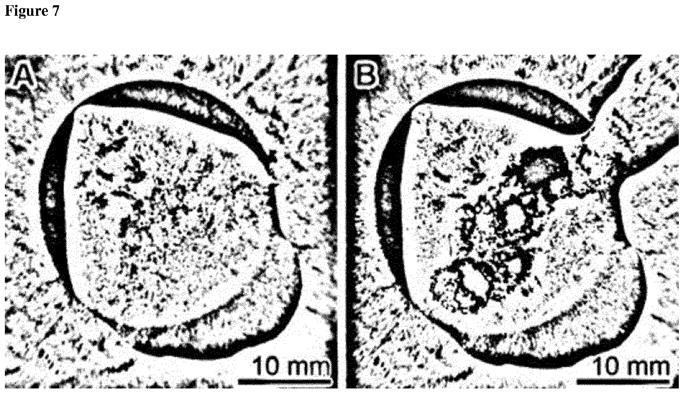

**Figure 7** shows the gel-formation of polyP with hyaluronic acid in the presence of Mg²⁺. (**A**) Mixing of 50 mg/0.5 ml of Na-polyP with 50 mg/0.5 ml of hyaluronic acid resulted in a clear solution. **(B)** If Mg²⁺ ions (1 ml of 50 mg MgCl₂•6 H₂O/ml) are added extensive flakes are formed.

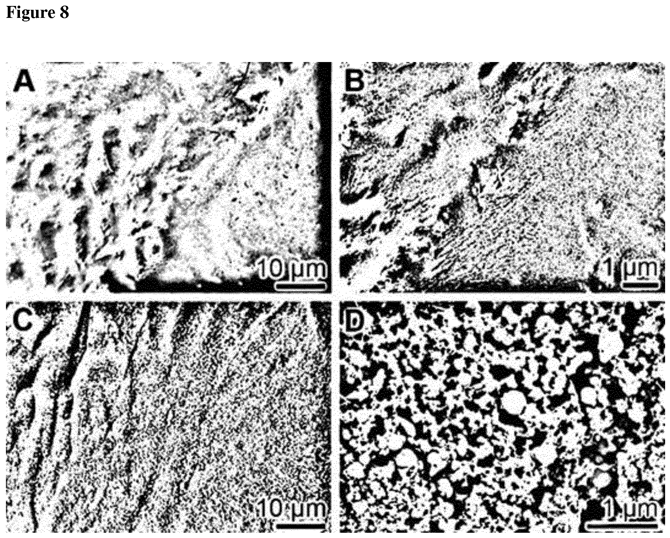

**Figure 8** shows the SEM analysis of HA-polyP. (**A** and **B)** Analysis of a HA-polyP layer without addition of MgCl₂. **(C** and **D)** Visualization of the microparticles present in the flakes, formed from hyaluronic acid and Na-polyP after addition of MgCl₂.

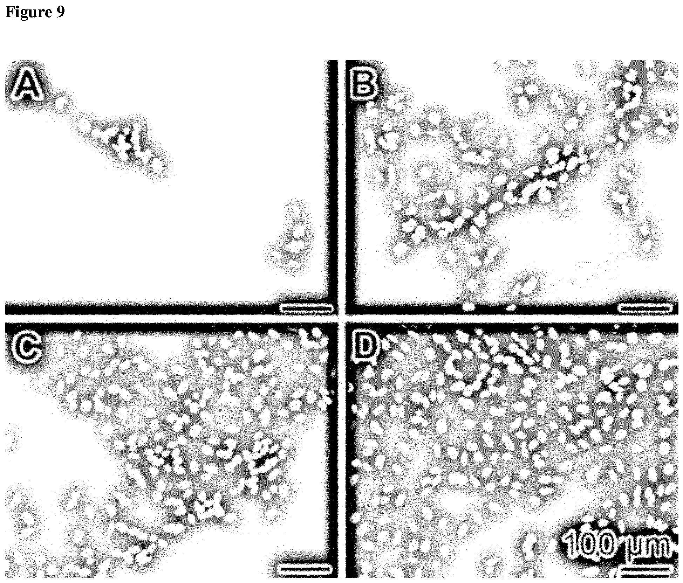

**Figure 9** shows the attachment of chondrocytes to (**A**) an untreated glass slide, **(B)** a HA-treated slide, and **(C** and **D)** a HA-Mg²⁺-Na-polyP-coated slide. The cells were cultivated onto the slides for 1 h (C) or 3 h (A, B and D). The nuclei of the cells were stained with DAPI.

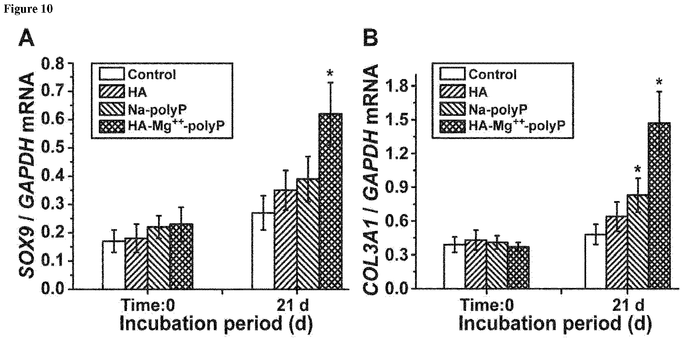

**Figure 10** shows the changes of transcript levels of *SOX9 and COL3A1* in chondrocytes during a 21 d incubation period. The cells were cultivated either in the absence of additional components (open bars), in the presence of Na-polyP (hatched to the right) or onto either hyaluronic acid (HA) coated slides (hatched to the left) or on HA-Mg²⁺-Na-polyP matrices (filled bars). After incubation, the RNA was extracted from the cells and then subjected to qRT-PCR. The *GAPDH* expression level was chosen to normalize the transcript levels for *SOX9* and *COL3A1.* Standard errors of the means (SEM) are indicated (n = five experiments per time point); * *P* < 0.05.

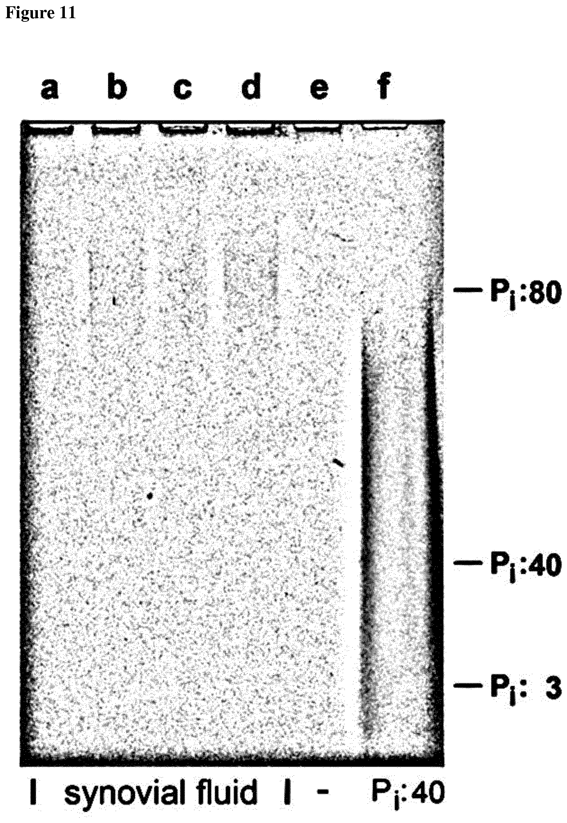

**Figure 11** shows the abundance and size of polyP, isolated from synovial fluid. Different concentrations of extracts from the synovial fluid were applied per lane; extracts from 10 ml of fluid **(lane a),** from 15 ml **(b** and **c)** and 20 ml **(d). Lane e** remained empty (-) and **f** was loaded with 20 µg of polyP₄₀ (Pᵢ:40). Size markers of polyP₈₀, polyP₄₀ and polyP₃ were run in parallel.

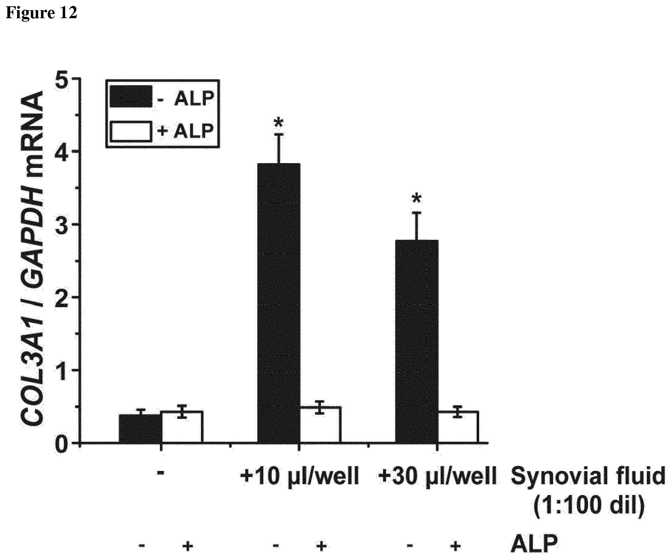

**Figure 12** shows the upregulation of *COL3A1* gene expression by synovial fluid. The chondrocytes remained either unexposed to the synovial fluid (-) or were incubated with two different concentrations of the fluid (at a dilution of 1:100), with 10 µl/well or 30 µl/well. The synovial fluid (or saline in controls) remained either untreated with ALP (-) or was pre-incubated with the enzyme (+) as described under "Methods". The means ± standard errors from five experiments per time point are shown; * *P* < 0.05.

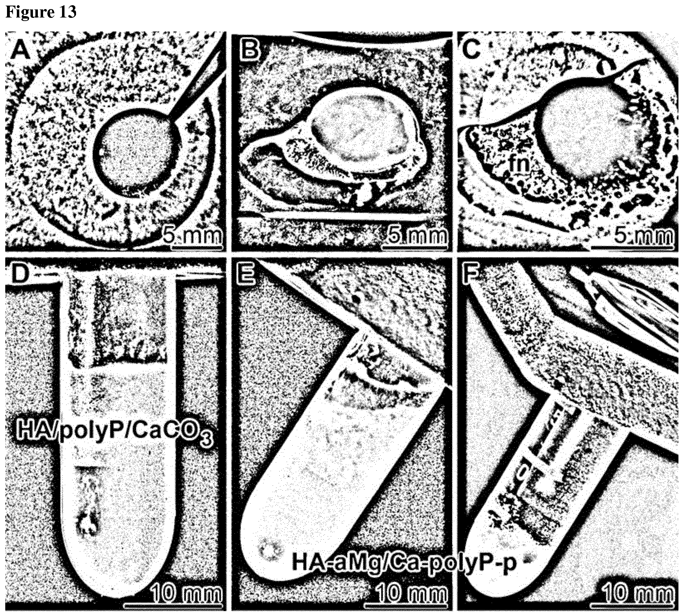

**Figure 13** shows the inventive hyaluronic acid-Mg/Ca-phosphate fluffy-microporous paste (HA-aMg/Ca-polyP-p). (**A**, **B** and **D)** A soluble hyaluronic acid (HA)/Na-polyP solution was prepared to which solid CaCO₃ was added (HA/polyP/CaCO₃). After addition of MgCl₂ **(C** and **E, F)** a fibrillar net starts to be formed at the rim of the suspension, reflecting the dissolution of the solid CaCO₃ salt particles. Simultaneously a paste is formed composed of hyaluronic acid, polyP, Mg²⁺ and Ca²⁺ (HA-aMg/Ca-polyP-p).

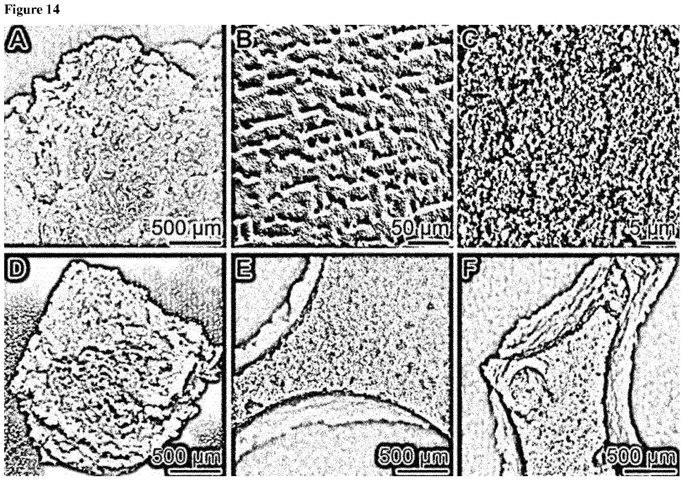

**Figure 14** shows the morphology of the HA-aMg/Ca-polyP-p paste. (**A** and **B,** and **D-F)** Digital light microscopic inspection of the HA-aMg/Ca-polyP-p prior (A and B) and after freeze-drying (D). In a separate series of experiments 5 mM CaCl₂ was added to HA-aMg/Ca-polyP-p; view before (E) and after freeze-drying (F). **(C)** SEM image showing the globular particles in HA-aMg/Ca-polyP-p that are glued together.

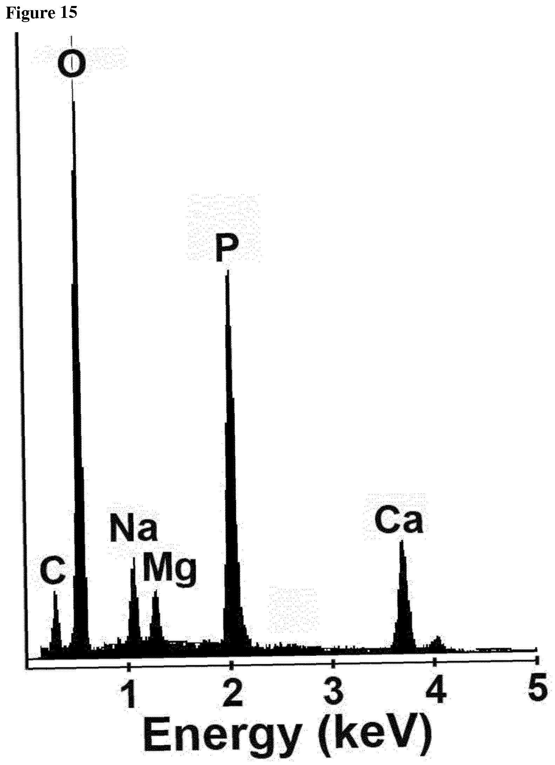

**Figure 15** shows the EDX analysis of HA-aMg/Ca-polyP-p. The prominent element peaks (O, Na, Mg, P and Ca) are marked.

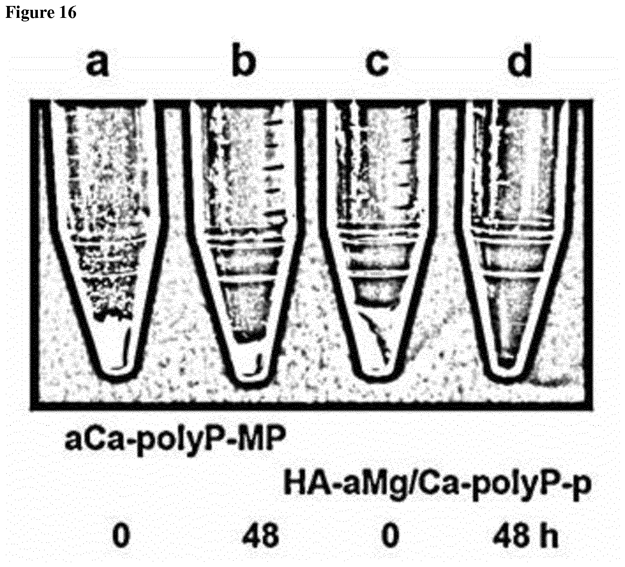

**Figure 16** shows the dissolution study of aCa-polyP-MP **(lanes a** and **b)** and of HA-aMg/Ca-polyP-p **(lanes c** and **d).** The microparticles were dissolved in Tris-HCl buffer (pH 8.0) and incubated for 0 h (lanes a and c) or 48 h (lanes b and d) in the presence of ALP.

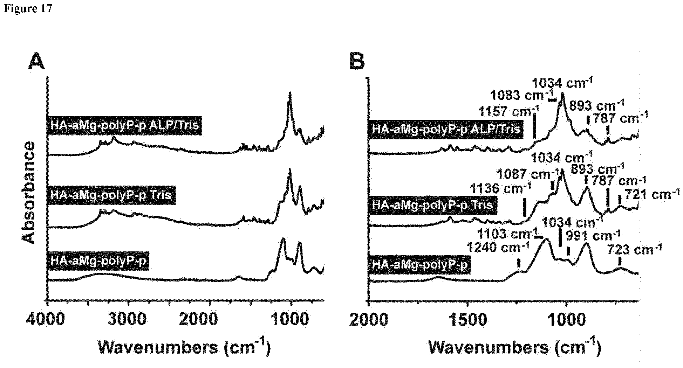

**Figure 17** shows the enzymatic hydrolysis of HA-aMg-polyP-p by ALP, as determined by FTIR spectroscopy. The spectra before (HA-aMg-polyP-p) and after transfer to Tris-HCl buffer (HA-aMg-polyP-p Tris) were determined. Finally, the FTIR spectrum was recorded after a 14 h incubation period in the presence of the ALP (HA-aMg-polyP-p ALP/Tris).

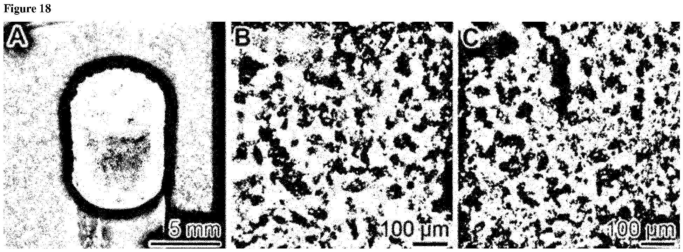

**Figure 18** shows the preparation of cylindrical blocks for subsequent biomechanical studies. (**A**) One cylinder is depicted. The light microscopic inspection revealed that the surfaces of the cylinders **(B)** comprise smaller channel pores (≈15 µm) compared to those that are found in the internal blocks with ≈25 µm **(C).**

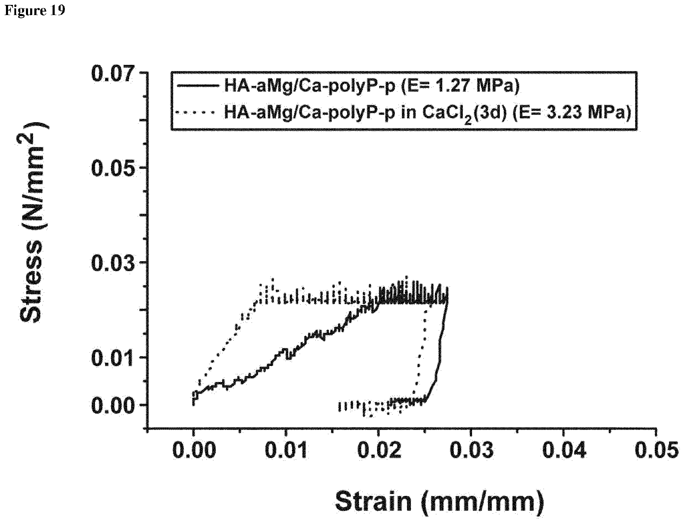

**Figure 19** shows the stress-strain curves for the HA-aMg/Ca-polyP-p scaffolds before (------) and after incubation with 5 mM CaCl₂ for 3 d (- - - -).

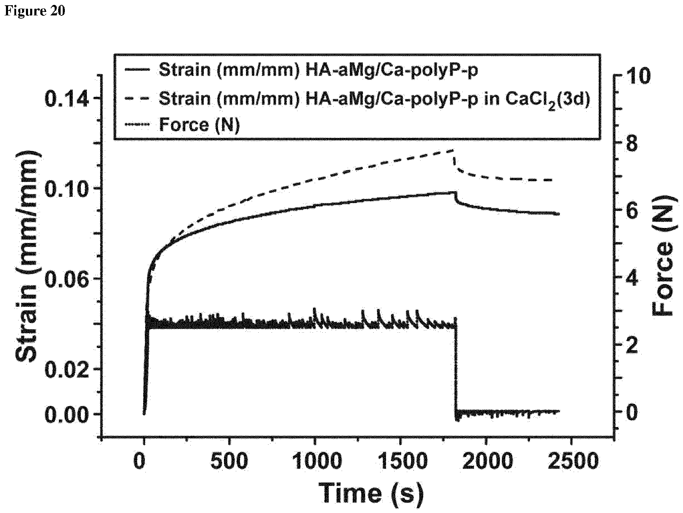

**Figure 20** shows the creep behavior of the HA-aMg/Ca-polyP-p material prior (------) and after a 5 mM CaCl₂ incubation for 3 d (- - - -).

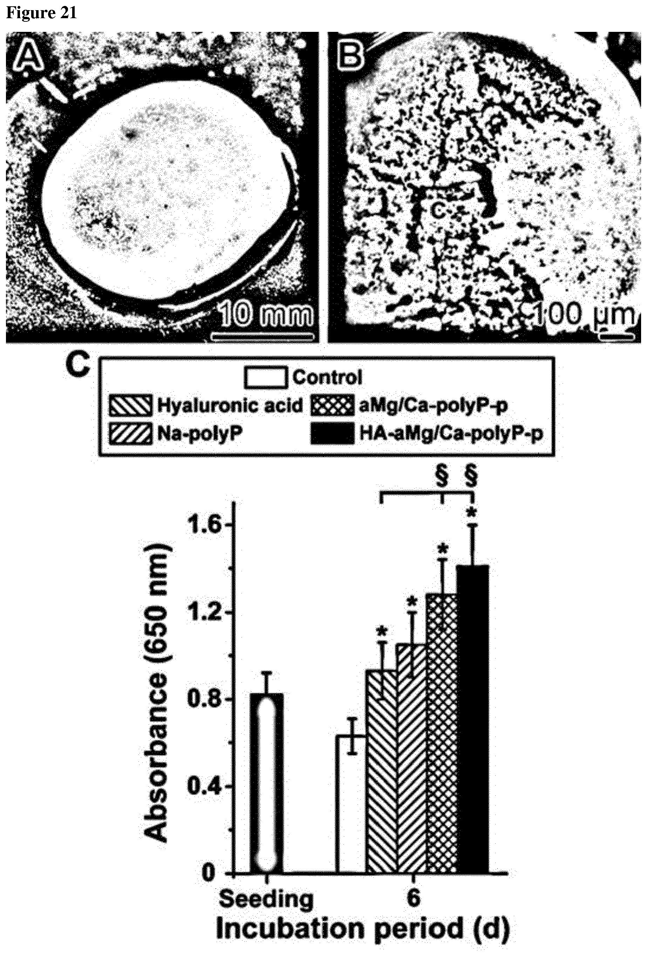

**Figure 21** shows the cell metabolic activity/growth of chondrocytes onto the inventive materials. (**A**) One piece of HA-aMg/Ca-polyP-p in the absence of cells. **(B)** A HA-aMg/Ca-polyP-p layer was placed into a chondrocyte medium/serum suspension and incubated for 24 h. Then the sample was removed and stained with haematoxylin-eosin stains; a homogenous cell layer **(c)** appears. **(C)** MTT assay result of the viability/growth studies with the additional components after cultivation for 6 d in the absence of any additional component (control; open bars) or in the presence of hyaluronic acid (hatched to the right), Na-polyP (hatched to the left), aMg/Ca-polyP-p (cross-hatched) and HA-aMg/Ca-polyP-p (filled). The absorbance value at time zero is likewise given (grey bar). Standard errors of the means (SEM) are indicated (n = 10 experiments per time point). * *P* < 0.05, with respect to the values of the controls after the 6 d incubation period; ^{§} *P* < 0.05 compared to the viability of the cells incubated with hyaluronic acid.

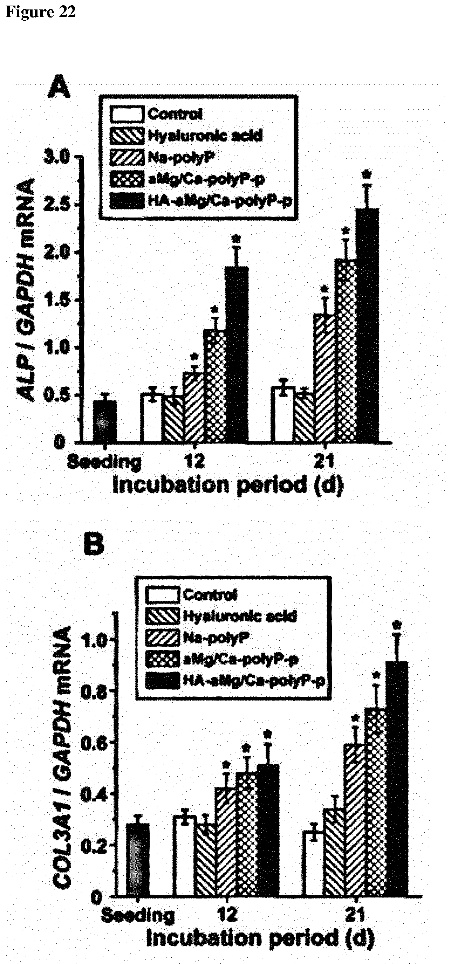

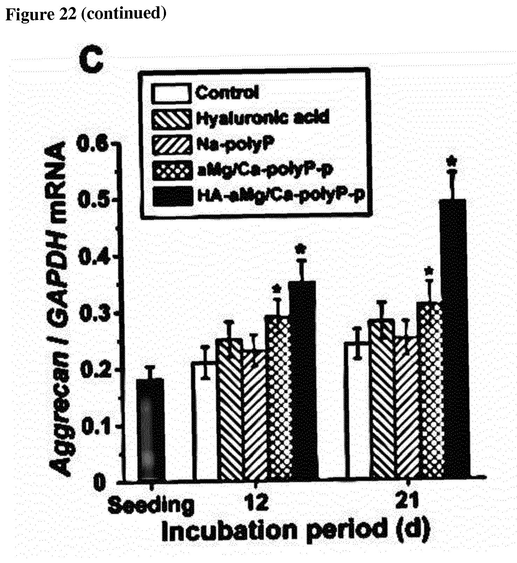

**Figure 22** shows the steady-state expression levels of the (**A**) *ALP* gene, **(B)** *collagen type III alpha 1* (*COL3A1*) gene as well as the **(C)** *aggrecan gene* in chondrocytes. The cells were incubated for 12 d or 21 d in the absence of the components (open bars), or the presence of 0.5 mg/ml hyaluronic acid (hatched to the right), 50 µg/ml of Na-polyP (hatched to the left), of aMg/Ca-polyP-p (cross-hatched) or of HA-aMg/Ca-polyP-p (filled). After the incubation the cells were harvested, their RNA extracted and subjected to qRT-PCR analyses. The expression values are given as ratios to the reference gene GAPDH; the ratios at time zero are in grey. The results are means from 5 parallel experiments; * *P* < 0.01; the values are computed against the expression measured in cells during seeding.

### Examples

In the following examples, the inventive method is described for polyP molecules with a chain length of 40 phosphate units. Similar results can be readily obtained by using polyP molecules with lower and higher chain lengths as disclosed herein.

### Materials and Methods

The Na-polyP of an average chain of 40 phosphate units used in the Examples was obtained from Chemische Fabrik Budenheim (Budenheim; Germany). Hyaluronic acid (HA; sodium salt from rooster comb) has been obtained from Sigma.

### Preparation of polyP microparticles

The fabrication of amorphous Mg-polyP microparticles (aMg-polyP-MP) was performed as follows. 3.86 g of MgCl₂ • 6H₂O were dissolved in 25 ml of distilled water and added dropwise to 1 g of Na-polyP in 25 ml distilled water at room temperature. The suspension, kept at pH 10.0, was stirred for 12 h. The microparticles formed were collected by filtration and washing with ethanol. Then, the microparticles were dried at 50°C.

The amorphous Ca-polyP microparticles (aCa-polyP-MP) were prepared as described (Muller WEG, et al. (2015) A new polyphosphate calcium material with morphogenetic activity. Materials Letters 148: 163-166; Müller WEG, et al. (2015) Retinol encapsulated into amorphous Ca2+ polyphosphate nanospheres acts synergistically in MC3T3-E1 cells. Eur J Pharm Biopharm 93: 214-223).

### Preparation of hyaluronic acid-Mg/Ca-phosphate fluffy-microporous paste

The production of hyaluronic acid-Mg/Ca-polyP paste (HA-aMg/Ca-polyP-p) was performed according to the general description given previously for amorphous Ca-polyP microparticles (aCa-polyP-MP) (Muller WEG, et al. (2015) A new polyphosphate calcium material with morphogenetic activity. Mater Lett 148: 163-166). In detail, 1 g of hyaluronic acid was dissolved in 10 ml of distilled water and supplemented with 1 g of solid Na-polyP. Subsequently, 0.5 g of solid CaCO₃ was added. To the resulting viscous hyaluronic acid/CaCO₃/polyP suspension 1.5 g of solid MgCl₂ • 6H₂O in 5 ml of distilled water was added. Continuation of mixing allows the formation of a white paste, designated as HA-aMg/Ca-polyP-p. After a 2 h period white flakes/deposits are formed.

In one series of experiments, the hyaluronic acid component was omitted from the polyP/CaCO₃/MgCl₂ fabrication process; the resulting paste was designated as aMg/Ca-polyP-p. To investigate the change of the properties with respect to the exposure to Ca²⁺ the HA-aMg/Ca-polyP-p was incubated with 5 mM CaCl₂ for 3 d.

### Microscopic inspections

Digital light microscopy can be performed, for example, with a VHX-600 Digital Microscope (Keyence) equipped with a VH-Z100zoom lens. Scanning electron microscopic (SEM) visualization can be performed, for example, using a HITACHI SU 8000 electron microscope (Hitachi High-Technologies Europe GmbH), or, e.g., Nova 600 Nanolab (FEI) is used.

### X-ray diffraction, energy-dispersive X-ray, and Fourier transformed infrared spectroscopic analyses

X-ray diffraction (XRD) experiments can be performed as described (Raynaud S, et al. (2002) Calcium phosphate apatites with variable Ca/P atomic ratio I. Synthesis, characterisation and thermal stability of powders. Biomaterials 23: 1065-1072). The patterns can be registered, for example, on a Philips PW 1820 diffractometer with CuKα radiation (λ = 1.5418 Å, 40 kV, 30 mA) in the range 2θ = 5-65° (Δ2θ = 0.02, Δt = 5 s).

Fourier transformed infrared spectroscopic (FTIR) analyses can be performed, for example, with micro-milled powder in an ATR (attenuated total reflectance)-FTIR spectroscope/Varian 660-IR spectrometer (Agilent), fitted with a Golden Gate ATR unit (Specac). For one series of experiments the paste formed from hyaluronic acid, Na-polyP and solid MgCl₂ is used. No CaCO₃ is added to avoid any superimpositions; the hyaluronic acid-Mg-phosphate paste is termed HA-aMg-polyP-p.

For energy-dispersive X-ray (EDX) spectroscopy an EDAX Genesis EDX System attached to the scanning electron microscope (for example, Nova 600 Nanolab) and operating at 10 kV with a collection time of 30-45 s can be used.

The inductively coupled plasma mass spectrometry analyses (ICP-MS) can be performed, for example, with an Agilent 7500 Series ICP-MS quadrupole system (Santa Clara; CA).

### Dissolution of HA-aMg-polyP-p by ALP

For the dissolution studies of 100 mg of either HA-aMg-polyP-p (or HA-aMg/Ca-polyP-p) or aCa-polyP-MP are suspended in 10 ml of 20 mM Tris-HCl buffer (pH 8.0). The aCa-polyP-MP particles were prepared as described (Muller WEG, et al. (2015) A new polyphosphate calcium material with morphogenetic activity. Mater Lett 148: 163-166). Then, recombinant bovine ALP, expressed in *Pichia pastoris* (e.g., from Sigma) is added at a final concentration of 20 units/ml. After an incubation period of 14 h or 48 h (at room temperature) the remaining pellet is analyzed by eye inspection and FTIR.

### Coating of well plates with polyP-Mg²⁺-HA-Mg²⁺

Each well of 24-well plates was filled with 0.5 ml of saline supplemented with 10% [w/v] hyaluronic acid (HA), and with 0.5 ml of saline containing 10% [w/v] Na-polyP. Subsequently, 1 ml of MgCl₂ • 6H₂O (50 mg/ml saline) was added, through which the gelation/ionic cross-linking reaction was initiated. The obtained HA-hydrogel, Mg²⁺-Na-polyP, is allowed to stand for 1 h to assure complete gelation (at room temperature). The fluid, released from the hydrogel, was removed carefully with a pipette. In control, the wells are either supplemented only with hyaluronic acid (50 mg HA/0.5 ml) or with Na-polyP (50 mg Na-polyP/0.5 ml). Most of the fluffs formed in the HA/Na-polyP assay attach to the bottom of the well plates. After standing at 37°C for 4 h, the fluid supernatant was aspirated and removed; then 1.5 ml of medium/FBS are added. Cells (10⁴ well) were added, and the cultures inspected after 1 h or 3 h of incubation after staining with DAPI (4',6-diamidino-2-phenylindole). The slices were inspected with an Olympus AHBT3 microscope.

### Human chondrocytes

For the experiments human chondrocytes, isolated from human knee, were used; for example, purchased from Lonza GmbH (Köln; Germany; NHAC-kn). They were cultivated in Chondrocyte Growth Medium (CGM BulletKit from Lonza) which contains growth factors and 5% fetal bovine serum (FBS). The cells were subcultured when they reached 80%-90% confluency. They were seeded with a density of about 10,000 cells per cm² and cell number duplicates about every 60 h. For the cell growth as well as the qRT-PCR experiments, the cultures were incubated for 6 d or 12 d/21 d, respectively.

To determine the growth rate of the cells, the chondrocytes were seeded into 6-well plates and cultured for 6 d in medium/serum. The following components were added separately to the cultures; 100 µg/ml of hyaluronic acid or 50 µg/ml either of Na-polyP (complexed stoichiometrically with Ca²⁺ [molar ratio of 2 phosphate monomers to 1 Ca²⁺] (Muller WEG, et al. (2011) Inorganic polymeric phosphate/polyphosphate as an inducer of alkaline phosphatase and a modulator of intracellular Ca2+ level in osteoblasts (SaOS-2 cells) in vitro. Acta Biomaterialia 7: 2661-2671)], aMg/Ca-polyP-p, or of HA-aMg/Ca-polyP-p. After termination, the cells were incubated with 3-[4,5-dimethyl thiazole-2-yl]-2,5-diphenyl tetrazolium (MTT) as described (Muller WEG, et al. (2015) A new printable and durable N,O-carboxymethyl chitosan-Ca2+-polyphosphate complex with morphogenetic activity. J Mat Chem B 3: 1722-1730). Subsequently, the remaining MTT dye was aspirated, and 200 ml of DMSO were added to solubilize the formazan crystals. Finally, the optical densities (OD) were determined at 650 nm. In one series of experiments, chondrocyte layers were stained with haematoxylin-eosin stain (Sigma).

### Identification of polyP in the synovial fluid

For a qualitative analysis, 20 ml of freshly taken synovial fluid were used for the extraction of polyP, which can be performed as described (Clark JE, et al. (1986) Isolation of intact chains of polyphosphate from "Propionibacterium shermanii" grown on glucose or lactate. J Bacteriol 168: 1212-1219; Imsiecke G, et al. (1996) Inorganic polyphosphates in the developing freshwater sponge Ephydatia muelleri: Effect of stress by polluted waters. Environ Toxicol Chem 15: 1329-1334). The polyP fraction obtained was applied onto a 7 M urea/16.5% polyacrylamide gel and stained with o-toluidine blue. PolyP standards of defined chain lengths were run in parallel (Lorenz B, Schröder HC (2001) Mammalian intestinal alkaline phosphatase acts as highly active exopolyphosphatase. Biochim Biophys Acta 1547: 254-261). Where indicated, the synovial fluid was pre-incubated with 20 units of ALP/ml synovial fluid. The recombinant bovine ALP, expressed in *Pichia pastoris* (e.g., from Sigma) can be used; incubation was performed for 6 h at room temperature prior to addition of the synovial fluid to the chondrocytes.

### Gene expression studies

The technique of quantitative real-time reverse transcription polymerase chain reaction (qRT-PCR) was applied to semi-quantitatively determine the effect of the polyP samples on chondrocytes. The cells were incubated for 0 d or 21 d in the absence of polyP or in the presence of 50 µg/ml of Na-polyP or onto slides, that were coated either with 300 µl of hyaluronic acid (10 mg/ml saline supplemented with 5 mM MgCl₂) or 300 µl of hyaluronic acid/Na-polyP (10 mg of hyaluronic acid/10 mg of Na-polyP in 5 mM MgCl₂).

In a further set of experiments, the cells were incubated for 12 d or 21 d either in the absence of any additional component besides of medium/serum, or in the presence of 0.5 mg/ml of hyaluronic acid, 50 µg/ml of Na-polyP (complexed with Ca²⁺ at a molar ratio of 2 [with respect to phosphate monomer] to 1 [Ca²⁺]; Muller WEG, et al. (2011) Inorganic polymeric phosphate/polyphosphate as an inducer of alkaline phosphatase and a modulator of intracellular Ca2+ level in osteoblasts (SaOS-2 cells) in vitro. Acta Biomater 7: 2661-2671), 50 µg/ml of aMg/Ca-polyP-p or of HA-aMg/Ca-polyP-p.

In one series of experiments, chondrocytes were incubated with synovial fluid (1:100 dilution) for 21 d. In order to remove polyp as present in the synovial fluid, the samples were pre-incubated with ALP, as described above.

The following genes were selected to determine the activation state of the human chondrocytes in culture; *ALP* (alkaline phosphatase; NM_000478.4) Fwd: 5'-TGCAGTACGAGCTGAACAGGAACA-3' (nt₁₁₄₁ to nt₁₁₆₄) (SEQ ID NO: 1) and Rev: 5'-TCCAC CAAATGTGAAGACGTGGGA-3' (nt₁₄₁₈ to nt₁₃₉₅; 278 bp) (SEQ ID NO: 2), *SOX9 SRY-box 9* (NM_000346) with the primer pair Fwd: 5'-TGCTGCTGGGAAACATTTGCAC-3' (nt₂₇₆₂ to nt₂₇₈₃) (SEQ ID NO: 3) and Rev: 5'-GGGCACTTATTGGCTGCTGAAAC-3' (nt₂₉₀₁ to nt₂₈₇₉; 140 bp) (SEQ ID NO: 4), *COL3A1* (collagen type III alpha 1; NM_000090) with the primer pair Fwd: 5'-ATTCCTTCGACTTCTCTCCAGCC-3' (nt₄₁₈₂ to nt₄₂₀₄) (SEQ ID NO: 5) and Rev: 5'-GTGTTTCGTGCAACCATCCTCC-3' (nt₄₃₇₇ to nt₄₃₅₆; 196 bp) (SEQ ID NO: 6), and *ACAN* (aggrecan transcript variant 1; NM_001135.3) Fwd: 5'-CCTCTGCATTCCACGAAGCTAACC-3' (nt₆₇₇₂ to nt₆₇₉₅) (SEQ ID NO: 7) and Rev: 5'-TGCCTCTGTCCCCACATCACTG-3' (nt₆₉₁₇ to nt₆₈₉₆; 146 bp) (SEQ ID NO: 8). The expression levels of these transcripts were correlated to the reference housekeeping gene GAPDH (glyceraldehyde 3-phosphate dehydrogenase, NM_002046.3) Fwd: 5'-ACTTTGTGAAGCTCATTTCCTGGTA-3' (nt₁₀₁₉ to nt₁₀₄₃) (SEQ ID NO: 9) and Rev: 5'-TTGCTGGGGCTGGTGGTCCA-3' (nt₁₁₃₆ to nt₁₁₁₇; 118 bp) (SEQ ID NO: 10). The qRT-PCR experiments are performed, for example, in an iCycler (Bio-Rad); the mean *Cₜ* values and efficiencies are calculated with the iCycler software (Bio-Rad); the estimated PCR efficiencies range between 93% and 103%.

### Determination of the biomechanical properties of the material

The overall biomechanical properties (the bulk Young's modulus) of the Mg-polyP-based cartilage scaffolds was determined using standardized cylindrical material samples measuring 5.4 mm in diameter and 4-5 mm in length, using, for example, the "MultiTest 2.5-xt Force Testing System" fitted with a 100 N Load Cell Unit (Mecmesin Ltd.). The data were recorded at a continuous frequency of 50 Hz using, for example, the Emperor XT Force software (Mecmesin Ltd.). A force of 0.5 N was used to determine the unconfined compressive strength; during the measurements it was kept constant for 60 s. Subsequently, an unloading period (0 N) follows for 300 s. From the force and displacement data as obtained, the bulk Young's modulus was calculated. The following equation is used: E [Young's modulus] equals to σ [stress]/ε [strain], whereby σ = force (N)/area (mm²) and ε = ΔL [difference in length] (mm)/L₀ [initial length] (mm). For the recording of the creep-recovery-curves, a constant load of 2.5 N was applied to the samples and held for 1800 s. Subsequently, the recovery period at 0 N follows which is limited to 600 s. The data for the creep-recovery-curves were recorded at a frequency of 50 Hz and again used to calculate the strain (ε) for the preparation of the strain-time graphs.

### Water content

The two scaffold forms, HA-aMg/Ca-polyP-p and HA-aMg/Ca-polyP-p-treated with 5 mM CaCl₂ for 3 d, were cut to slices to approximately the same dimensions. After removal of the superficial, oozing liquid the samples were freeze-dried overnight (e.g., Sentry 2.0 lyophiliser; SP Scientific). Subsequently, the dry weight was determined and the % water content calculated.

### Statistical analysis

After finding that the respective values followed a standard normal Gaussian distribution and that the variances of the respective groups were equal, the results were statistically evaluated using the independent two-sample Student's *t*-test.

### Results

### Production of polyP microparticles and their characterization

As outlined above, microparticles were prepared from Ca-polyP as well as from Mg-polyP. The size range of the aCa-polyP-MP varied between 220 and 535 nm (average 352±122 nm) (Figure 3A and C), while the dimensions of the aMg-polyP-MP were slightly smaller with 130 to 317 nm (average of 245±138 nm) (Figure 3B and D).

Element analysis of the polyP microparticles was performed by EDX. The spectra showed for the aCa-polyP-MP pronounced peaks for Ca, P and O, while only minor signals appeared for C and Na (Figure 4A). The pattern for aMg-polyP-MP showed distinct peaks for Mg, P and O and again only small ones for C and Na (Figure 4B). A quantitative analysis of the particles was performed by ICP-MS. The final divalent cation/P atomic ratio for the obtained aCa-polyP-MP was 0.93±0.12 and for aMg-polyP-MP 0.81±0.09.

The FTIR spectra obtained from microparticles, aCa-polyP-MP and aMg-polyP-MP, showed strong IR absorption bands in the low and high frequency regions, due to phosphate moieties and the OH groups (Figure 5). The bands recorded could be attributed to the vibrations of the following structural units: The peaks at ∼ 3400 cm⁻¹ and 1627 cm⁻¹ are mainly assigned to the OH stretching and bending vibrations of absorbed water. The band near 1261 cm⁻¹ could be designated to the asymmetric stretching of the doubly bonded oxygen vibration, i.e. *v*ₐₛ(PO₂)⁻. The weaker band at 1196 cm⁻¹ (not marked in Figure 5) reflects the PO₂ symmetric stretching mode *v*ₛ(PO₂)²⁻. The absorption bands close to 1083 cm⁻¹ and 999 cm⁻¹ are assigned to the asymmetric and symmetric stretching modes of chain-terminating PO₃ groups (*v*ₐₛ(PO₃)²⁻ and *v*ₛ(PO₃)²⁻). The absorption band near 864 cm⁻¹ is assigned to the asymmetric stretching modes of the P-O-P linkages, *v*ₐₛ(P-O-P) and the partially split band is centered around 763 cm⁻¹ and is assigned to the symmetric stretching modes of these linkages, the *v*ₛ(P-O-P). A comparison between the spectra of Na-polyP, Ca-polyP and Mg-polyP shows the common feature for polyP in the 1300-600 cm⁻¹ region. Those signals reflect the polyP backbones and are not broken down during the reaction with the Ca²⁺ or Mg²⁺. However, the peaks shifted in the Ca-polyP and Mg-polyP samples, if compared to those in Na-polyP. These changes in the phosphate structures are attributed to the Ca²⁺ or Mg²⁺ ions, which generally provide ionic cross-linking between non-bridging oxygen of the phosphates groups. Those Ca²⁺ and Mg²⁺-caused cross-links further increased the interacting bond strength between adjacent polyP chains. Additionally, the modification in the polyP structures can be understood further on the basis of the changes in the respective terminal charge densities at an anionic site, i.e at the sites of formation of P-O-*M* (where *M* is Ca²⁺ or Mg²⁺; it is in Na-polyP at 1261 cm⁻¹, in Ca-polyP at 1240 cm⁻¹ and Mg-polyP at 1243 cm⁻¹). Thus, IR spectra confirmed the ionic interactions between the two divalent cations and the respective polyP chains formed within Ca-polyP and Mg-polyP.

Figure 6 shows the XRD patterns of Na-polyP, Ca-polyP and Mg-polyP powder samples. While Na-polyP was used as an unchanged salt sample, microparticles were prepared from Ca-polyP and Mg-polyP. All these samples were obtained after drying at 60°C for 24 h. The absence of any sharp peak confirmed the absence of any crystalline phase, qualifying the microparticles as amorphous (aCa-polyP-MP and aMg-polyP-MP, resp.). The distinct amorphous phase is shown within the broad peak from 20° to 40° (theta) degrees for Na-polyP, and for Ca-polyP and Mg-polyP around 30° (theta) degrees.

### In situ formation of Mg-polyP microparticles and their interaction with HA

The formation of Mg-polyP microparticles from Na-polyP and hyaluronic acid was processed in 24-well plates, starting from 50 mg/0.5 ml of Na-polyP and 50 mg/0.5 ml of hyaluronic acid in saline. This sample appeared as a perfect solution (Figure 7A). If MgCl₂•6 H₂O is added, an almost immediate gelation process starts that is visualized by an intense streak formation (Figure 7B). The gel flakes as formed attach to the glass slides and allowed an inspection by SEM (Figure 8). Those aspects revealed that the microparticles formed from Na-polyP and hyaluronic acid and after addition of Mg²⁺ appeared as almost perfect spherical microparticles with an average size of 389±143 nm (Figure 8C and D). In contrast, hyaluronic acid flakes that attached onto the slides and produced from a solution of hyaluronic acid in saline without Mg²⁺ revealed only a quite smooth coat (Figure 8A and B). In assays with Na-polyP no flakes could be visualized after addition of Mg²⁺.

### Attachment of human chondrocytes onto HA-Mg^{2±}-polyP-covered slides

The propensity of chondrocytes to attach to differentially covered glass slides was inspected 3 h after transfer of the cells onto the slides. The cells were visualized by staining with DAPI. After irradiation with fluorescent light it becomes evident that only a few cells attached to untreated glass surfaces after 3 h (Figure 9A), while significantly more cells were visualized after 3 h on surfaces coated with hyaluronic acid (Figure 9B). Comparably intense is the attachment of chondrocytes to glass slides that had been coated with HA-Mg²⁺-Na-polyP (Figure 9C and D). Even after a short 1 h incubation period the cells abundantly attached to HA-Mg²⁺-Na-polyP-coated slides (Figure 9C), and even more cells were detected after 3 h incubation (Figure 9D).

### Expression of SOX9 and COL3A1 in response to polyP exposure

The inventor selected the genes of the transcription factor *SOX9* and of the collagen gene *COL3A1* as reliable markers for *in vitro* studies of chondrocytes with respect to cartilage differentiation. The inventor found that the transcript level of *SOX9* changes during the 21 d incubation only if the cells were incubated onto the HA-Mg²⁺-Na-polyP matrix (Figure 10A). While the expression of this gene does only slightly vary if the cells were cultured in the absence of any additional component or only moderately with Na-polyP or hyaluronic acid, an almost 3-fold increase of the expression level was observed if the cells grew onto the HA-Mg²⁺-Na-polyP surface.

In contrast, the expression level of *COL3A1* increases significantly during the 21 d incubation period by 2-fold (with respect to day 0) oder 1.7-fold (with respect to the control) if the chondrocytes were exposed to Na-polyP and even 5-fold (with respect to day 0) oder 3.1-fold (with respect to controls) if the cells were cultured onto HA-Mg²⁺-Na-polyP (Figure 10B), while only a slight increase is seen in assay with hyaluronic acid.

### Tracing polyP in synovial fluid

Applying the urea/polyacrylamide gel electrophoresis technique, polyP could be identified in the gel as a distinct band around the polyP marker size range of polyP₈₀ (Figure 11 lanes a-d); in this region no toluidine staining could be seen in the parallel lane (lane e), which did not contain any polyP. The intensity of Na-polyP (20 µg/slot) used for the preparation of the microparticles is seen in lane f.

### Effect of synovial fluid on the collagen gene (COL3A1) expression

To test the effect of human synovial fluid on the expression of collagen *COL3A1* gene, the chondrocytes were incubated for 21 d in the standard assay. A 1:100 dilution of the fluid was applied and added at a concentration of 10 µl/well or 30 µl/well (Figure 12). Using these conditions the expression of the gene strongly increases from the base level of 0.4 units with respect to the steady-state-expression of the reference gene GAPDH to 3.82 units at 10 µg/well or to 2.77 units at 30 µl/well. However, this strong stimulatory activity was completely abolished if the synovial fluid was pre-incubated with ALP, as described above.

### Production of hyaluronic acid-Mg/Ca-phosphate fluffy-microporous paste

The fabrication of hyaluronic acid-Mg/Ca-phosphate paste (HA-aMg/Ca-polyP-p) was performed by successive addition of solid, water-soluble Na-polyP to soluble hyaluronic acid, as described under "Methods". Then solid CaCO₃ was added which did not dissolve in the viscous hyaluronic acid/polyP solution (Figure 13A and B). After addition of solid MgCl₂ the paste started to dissolve at the rim under formation of a 500 µm sized fibrillar net (Figure 13C). This series of steps was microscopically followed in a one-cavity microscope slide. Eye-inspection of these steps in 1.5 ml Eppendorf tubes revealed that the particles, composed of hyaluronic acid/Na-polyP/CaCO₃ did not sediment under normal conditions (Figure 13D). After addition of MgCl₂, paste-like deposits are formed that sediment to the bottom of the tubes by forming the HA-aMg/Ca-polyP-p paste (Figure 13E and F).

The microscopic morphology of the paste, HA-aMg/Ca-polyP-p, was inspected by digital light microscopy as well as by SEM (Figure 14). At lower magnification, and before freeze-drying, the HA-aMg/Ca-polyP-p appeared as a fluffy scaffold (Figure 14A) with a shed-like lattice (Figure 14B). Visualization by SEM revealed globular particles ranging between 320 and 560 µm (Figure 14C). After freeze-drying the fluffy scaffold turns to a more solid deposit (Figure 14D). After addition of 5 mM CaCl₂ to HA-aMg/Ca-polyP-p (3 d) the material became more compact, as can be deduced before (Figure 14E) and after freeze-drying (Figure 14F). XRD analysis of the freeze-dried HA-aMg/Ca-polyP-p revealed no peak between a 2-theta range 5-50 degree (data not shown), indicating that the globular particles in HA-aMg/Ca-polyP-p are amorphous.

For the analysis of the elements, present in the HA-aMg/Ca-polyP-p, EDX spectroscopy was applied. As shown in Figure 15, the spectrum shows the characteristic signals for O, P, Na (remaining from the Na-polyP starting material) as well as of Mg and Ca both linked *via* ionic bonds to the polyP backbone, under formation of insoluble deposits. The element weight-% ratio, calculated from the EDX data analyses, was determined to be Ca:Na:Mg with 1:0.22:0.14.

### Dissolution of HA-aMg/Ca-polyP-p by ALP

In a comparative study, aCa-polyP-MP *versus* HA-aMg/Ca-polyP-p the materials were incubated with ALP, as described above. Even by eye inspection it becomes obvious that during the 48 h incubation period the aCa-polyP-MP particles undergo only a lower hydrolytic dissolution, if compared with HA-aMg/Ca-polyP-p (Figure 16). During this incubation period only about 48% (by dry weight) of the aCa-polyP-MP were transferred to a soluble form (Figure 16; lanes a and b). In contrast, (almost) the total amount of HA-aMg/Ca-polyP-p was solubilized in the presence of ALP (lanes c and d).

In order to analyze also the process of hydrolysis of HA-aMg-polyP-p (due to technical reasons this paste was used instead of HA-aMg/Ca-polyP-p) also qualitatively the sediments were dried and subjected to FTIR spectral analysis (Figure 17). The HA-aMg-polyP-p were either used directly for the analysis or were incubated in Tris-HCl buffer in the absence or presence of ALP for 14 h. The results of the non-incubated material show the main characteristic peaks for HA-aMg-polyP-p material; the peaks at the wavenumbers 1240 cm⁻¹ (symmetric stretching mode *v*ₛ(PO₂)²⁻), 1103 cm⁻¹ (*v*ₐₛ(PO₃)²⁻), 897 cm⁻¹ (*v*ₛ(P-O-P)) and 723 cm⁻¹ (*v*ₐₛ(P-O-P)), which indicate the polyP microstructure (Figure 17). These peaks remained almost unchanged during a 14 h incubation period in Tris-HCl buffer. However, after addition of ALP a significant shift of the bands occurred. Especially pronounced are the following shifts; increase of the absorbance bands in the region of 1100 cm⁻¹ to 980 cm⁻¹ due to P-O stretching vibrations from PO₄³⁻ vibrational mode of the phosphate group. While the polyP reflected by the band at 1240 cm⁻¹ disappeared and the intensity of peak at 897 cm⁻¹ was decreased and split into the two peaks at 913 cm⁻¹ and at 893 cm⁻¹, which is characteristic of the P-O stretching vibrations from PO₄³⁻ groups. The above results indicate that the HA-aMg-polyP-p have undergone hydrolytic degradation, induced by ALP.

### Morphology

For the required mechanical studies cylindrical blocks (5.4 mm [diameter] x 4-5 mm [length] were prepared (Figure 18A). The surface of those samples was porous with an average pore size of ≈15 µm (Figure 18B). Breaking the blocks revealed that the interior of the samples is traversed by larger channels with an average dimension of ≈25 µm (Figure 18C).

### Determination of the biomechanical properties of the material

Compression tests were performed using a tensile/compression test system to characterize the bulk mechanical properties of the polyP material. In order to study the effect of Ca²⁺ on the hardness/consistency of HA-aMg/Ca-polyP-p the material was incubated in the presence of 5 mM CaCl₂ for 3 d at ambient temperature.

Already the shapes of the stress-strain curves (Figure 19) indicate the change in the properties of HA-aMg/Ca-polyP-p after exposure to Ca²⁺. In the absence of the cation the polymer initially shows a prolonged deformation segment upon loading which also comprises discontinuities that reflect micro-crumblings of the material. After reaching the maximal stress of 0.025 MPa the loading stress was kept constant and a creep phase could be observed which is terminated with the beginning of the unloading phase. In the following the stress was kept constant at 0 MPa. The pronounced indentation to the left shows a partial recovery of the material and thus indicates some elastic/viscoelastic properties of HA-aMg/Ca-polyP-p. The Young's modulus which was calculated at the end of the loading phase amounts to 1.27 MPa.

In contrast to HA-aMg/Ca-polyP-p, the material exposed to CaCl₂ showed a faster stress increase which is reflected by a higher slope of the initial deformation. The increase of the curve is close to perfect linear indicating that no distinct micro-crumblings occur (Figure 19). The creep period is longer if compared to HA-aMg/Ca-polyP-p and likewise the final elastic/viscoelastic recovery period is also extended. The Young's modulus was considerably numerically higher and measures 3.23 MPa. This shift in the mechanical properties in response to Ca²⁺ was seen already after an exposure time of 60 min to CaCl₂. These differences between the material prior and after treatment with CaCl₂ was also reflected by the creep measurements using constant compressive stress for 30 min followed by a 10 min recovery time (Figure 20). The untreated HA-aMg/Ca-polyP-p shows a similar biphasic creep-recovery curve with incomplete recovery upon unloading compared to the material treated with CaCl₂. However, the Ca²⁺-treated material shows a more pronounced creep behavior. The initial strain response is faster and already reaches the maximal strain response after ≈ 5 s, with ≈ 0.23 mm/mm. Both samples were loaded with a force of 2.5 N.

The water content of the unprocessed HA-aMg/Ca-polyP-p was found to be 77.33±1.64%; in comparison the CaCl₂-treated samples contained slightly less water with 63.38±0.78%.

### Bio-compatibility of the surface to the HA-aMg/Ca-polyP-p material

If HA-aMg/Ca-polyP-p past pieces are formed by ionic gelation of the polymers (Figure 21 A) and put into a chondrocyte culture in medium/serum for 24 h a homogenous cell layer is formed on their surfaces (Figure 21B). The cell layer was visualized by haematoxylin-eosin color reaction.

For cell culture studies with the aim to assess the effect of the biomaterials on cell growth, the MTT assay was applied. The cells were cultivated either in the absence (control) or presence of the following components; 100 µg/ml of hyaluronic acid, or of 50 µg/ml either of Na-polyP, aMg/Ca-polyP-p, or of HA-aMg/Ca-polyP-p (Figure 21C). At time zero (seeding the cells) the absorbance at 650 nm was measured with 0.82±0.11 absorbance units. Incubating the cells in the absence of any additional component (controls) the metabolic activity drops significantly to 0.63±0.08 units, while all other assays which contained hyaluronic acid and/or polyP show a significant increase in the activity. The highest stimulation of growth was determined in the assays with HA-aMg/Ca-polyP-p (1.41±0.19) and aMg/Ca-polyP-p (1.28±0.16); the values are significantly higher if compared with the values measured for hyaluronic acid (0.93±0.13).

### Expression of the genes encoding for ALP, collagen type III alpha 1 and aggrecan in chondrocytes

The steady-state-expression of three determining genes for chondrocytes, *ALP*, *collagen type III alpha 1* (*COL3A1*) and *aggrecan* was selected for the determination of the morphogenetic activity using the qRT-PCR technique; the house-keeping gene *GAPDH* was used as a reference gene. Cells were incubated for 12 d or 21 d without any additional component (controls) or with 0.5 mg/ml of hyaluronic acid or 50 µg/ml either of Na-polyP, aMg/Ca-polyP-p or of HA-aMg/Ca-polyP-p (Figure 22). The response of the *ALP* gene expression was rapid and significant for all three polyP containing samples, already after the 12 d incubation (Figure 22A). The highest *ALP* expression levels were determined after 21 d for aMg/Ca-polyP-p (3.2-fold induction compared to the controls after the same incubation period) and HA-aMg/Ca-polyP-p (3.9-fold).

The expression profile for *COL3A1* is very much similar than the one measured in the *ALP* assays. Again the highest expression levels are seen after 21 d with 2.9-fold in the presence of aMg/Ca-polyP-p, and 3.8-fold for HA-aMg/Ca-polyP-p (Figure 22B). Under the experimental conditions used here, again no significant stimulatory effect was measured for hyaluronic acid.

The expression levels of the *aggrecan* gene was lover in chondrocytes compared to the ones of *ALP* and *COL3A1.* However, also using this gene as a marker the expression in assays with aMg/Ca-polyP-p and HA-aMg/Ca-polyP-p was significantly upregulated at both incubation time periods (Figure 22C). Again the highest expression level was measured for HA-aMg/Ca-polyP-p (2.1-fold) after the 21 d incubation.

## Claims

1. A method for producing a pharmaceutical composition comprising amorphous magnesium polyphosphate microparticles, comprising the steps of
i) dissolving a soluble polyphosphate salt in an aqueous medium,
ii) adding an aqueous solution of a soluble magnesium salt to said polyphosphate salt solution, and
iii) collecting and drying of the particles formed after washing with alcohol, such as ethanol.

2. A method for producing a pharmaceutical composition comprising amorphous magnesium polyphosphate microparticles and hyaluronic acid, comprising the steps of
i) mixing of an aqueous solution of a soluble polyphosphate salt with an aqueous solution of hyaluronic acid,
ii) adding a soluble magnesium salt to the mixture from step (i), and
iii) allowing said mixture to fully gelatinate.

3. The method according to claim 1 or 2, wherein said soluble polyphosphate salt is sodium polyphosphate.

4. The method according to any one of claims 1 to 3, wherein the chain length of said polyphosphate is between about 3 to about 1000 phosphate units, preferably between about 10 to about 100 phosphate units, and most preferred about 40 phosphate units.

5. The method according to any one of claims 1 to 4, wherein said magnesium salt is magnesium chloride.

6. The method according to any one of claims 1 to 5, wherein the amorphous magnesium polyphosphate microparticles are formed from sodium polyphosphate in the presence of magnesium chloride at a stoichiometric ratio of 0.1 to 20 (phosphate to magnesium), preferably of 1 to 4, and most preferably of 2.

7. The method according to any one of claims 1 to 6, wherein the magnesium polyphosphate material is obtained by addition of a solution containing between about 100 to 200 g of magnesium chloride hexahydrate to a solution containing between about 20 to 60 g/L of sodium polyphosphate at a ratio of approximately 1:1 (volume/volume; magnesium chloride solution to sodium polyphosphate solution).

8. The method according to any one of claims 2 to 7, comprising
i) admixing of about one volume of saline supplemented with about 10% [w/v] hyaluronic acid with about one volume of saline containing about 10% [w/v] Na-polyP,
ii) adding about two volumes of magnesium chloride hexahydrate (at 50 mg/ml in saline), in order to initiate the gelation/ionic cross-linking reaction, and
iii) allowing the obtained mixture to assure complete gelation.

9. The method according to any one of claims 2 to 8, further comprising the addition of a water-insoluble calcium salt or a calcium salt with low solubility in water to the mixture from step (i) before the addition of the magnesium salt, and continuation of mixing in order to allow for a hyaluronic acid-magnesium/calcium-polyphosphate paste formation.

10. The method according to claim 9, wherein the calcium salt is calcium carbonate.

11. The method according to any one of claims 2 to 10, wherein the production of the cartilage-like material comprises
i) preparing of an aqueous solution containing about 100 g/L of hyaluronic acid,
ii) adding about 100 g of solid sodium polyphosphate to about 1 L of the solution prepared in step (i),
iii) adding of about 50 g of solid calcium carbonate to about 1 L of the solution obtained in step (ii),
iv) adding of about 150 g of solid magnesium chloride hexahydrate, dissolved in about 500 ml of distilled water, to the viscous hyaluronic acid/calcium carbonate/polyphosphate suspension obtained in step (iii), and
v) continuous mixing for about 2 h allowing for a hyaluronic acid-magnesium/calcium-polyphosphate paste formation.

12. A pharmaceutical composition comprising amorphous magnesium polyphosphate microparticles, a gel, and/or a paste produced according to any one of claims 1 to 11 for use in medicine.

13. A pharmaceutical composition comprising amorphous magnesium polyphosphate microparticles, a gel, and/or a paste produced according to any one of claims 1 to 11 for use in the regeneration and repair of cartilage or cartilage-like bone material, or as a bonding material for cartilage and bone.

14. A pharmaceutical composition comprising amorphous magnesium polyphosphate microparticles, a gel, and/or a paste produced according to any one of claims 1 to 11 for use in the prevention and/or treatment of joint dysfunctions and osteoarthritis through scavenging of synovial fluid calcium ions implicated in crystal formation.


SEQUENCE LISTING

<110>  NanotecMARIN GmbH

<120>  AMOPRHOUS HYALURONIC ACID-MAGNESIUM/CALCIUM POLYPHOSPHATE
       MICROPARTICLES FOR CARTILAGE REGENERATION AND REPAIR

<130>  M33838EP

<150>    GB1612280.6
<151>    2016-07-15

<160>  10

<170>  PatentIn version 3.5

<210>  1
<211>  24
<212>  DNA
<213>  Homo sapiens

<400>  1
tgcagtacga gctgaacagg aaca                                              24


<210>  2
<211>  24
<212>  DNA
<213>  Homo sapiens

<400>  2
tccaccaaat gtgaagacgt ggga                                              24


<210>  3
<211>  22
<212>  DNA
<213>  Homo sapiens

<400>  3
tgctgctggg aaacatttgc ac                                                22


<210>  4
<211>  23
<212>  DNA
<213>  Homo sapiens

<400>  4
gggcacttat tggctgctga aac                                               23


<210>  5
<211>  23
<212>  DNA
<213>  Homo sapiens

<400>  5
attccttcga cttctctcca gcc                                               23


<210>  6
<211>  22

<212>  DNA
<213>  Homo sapiens

<400>  6
gtgtttcgtg caaccatcct cc                                                22


<210>  7
<211>  24
<212>  DNA
<213>  Homo sapiens

<400>  7
cctctgcatt ccacgaagct aacc                                              24


<210>  8
<211>  22
<212>  DNA
<213>  Homo sapiens

<400>  8
tgcctctgtc cccacatcac tg                                                22


<210>  9
<211>  25
<212>  DNA
<213>  Homo sapiens

<400>  9
actttgtgaa gctcatttcc tggta                                             25


<210>  10
<211>  20
<212>  DNA
<213>  Homo sapiens

<400>  10
ttgctggggc tggtggtcca                                                   20